(19) **Europäisches Patentamt**

**European Patent Office**

**Office européen des brevets**

(11) Veröffentlichungsnummer: **0 242 766 B1**

(12) **EUROPÄISCHE PATENTSCHRIFT**

(45) Veröffentlichungstag der Patentschrift: **18.03.92**

(51) Int. Cl.⁵: **A61F 13/15**

(21) Anmeldenummer: **87105459.9**

(22) Anmeldetag: **13.04.87**

(54) **Saugfähiger Wegwerfartikel.**

(30) Priorität: **17.04.86 DE 3613042**

(43) Veröffentlichungstag der Anmeldung:
**28.10.87 Patentblatt 87/44**

(45) Bekanntmachung des Hinweises auf die
Patenterteilung:
**18.03.92 Patentblatt 92/12**

(84) Benannte Vertragsstaaten:
**AT BE DE ES FR GB IT NL SE**

(56) Entgegenhaltungen:
**EP-A- 0 059 015**     **EP-A- 0 189 911**
**FR-A- 2 331 974**     **US-A- 3 215 578**
**US-A- 3 620 898**     **US-A- 3 844 288**
**US-A- 4 258 089**     **US-A- 4 405 397**

(73) Patentinhaber: **Paul Hartmann Aktiengesellschaft**
**Paul-Hartmann-Strasse**
**W-7920 Heidenheim(DE)**

(72) Erfinder: **Malowaniec, Krzysztof, Dipl.-Ing.**
**Nürtinger Weg 10**
**W-7920 Heidenheim(DE)**
Erfinder: **Simmler, Kurt**
**Bayernweg 23**
**W-7920 Heidenheim(DE)**

(74) Vertreter: **Becker, Maria, Dipl.-Phys.**
**Auf dem Haigst 29**
**W-7000 Stuttgart 70(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft einen saugfähigen Wegwerfartikel, wie insbesondere eine Windel nach dem Oberbegriff des Anspruchs 1.

Derartige Artikel sind beispielsweise bekannt aus DE-OS 19 14 179 und EP 00 59 015 B1.

In beiden Fällen sind als Sperrschichten einzelne Folien eingelegt, die entweder nur am Rande des Wegwerfartikels mit der porösen Auflageschicht verbunden sind (DE-OS 19 14 179) oder die zusätzlich zu einer Randbefestigung auch noch an weiteren, jeweils im Abstand voneinander angeordneten, über die gesamte Fläche der Sperrschicht verteilten Stellen mit der Auflageschicht verklebt sind (EP 0 059 015 B1).

Der Nachteil dieser Sperrschichten besteht darin, dass sie im Prinzip jeweils eine eigenständige Schicht darstellen, die lediglich an einzelnen Stellen mit der Auflageschicht verbunden sind. Daher müssen diese Sperrschichten u.a. eine Dicke aufweisen, die ihnen eine eigenständige ausreichende Festigkeit verleiht. Dadurch benötigen diese Sperrschichten relativ viel Material. Bei den gattungsgemässen Massenartikeln fällt dies bereits kostenmässig ins Gewicht. Auch sind diese Schichten beim Tragen unangenehm steif.

Es ist darüber hinaus auch bereits aus US-PS 3,799,167 bekannt, die betreffenden Sperrschichten am Rande eines saugfähigen Wegwerfartikels durch ein Tränken der porösen Auflageschicht mit einem flüssigkeitsundurchlässigen Material zu erzeugen. Der Nachteil dieser Ausführungsform besteht darin, dass wiederum ein relativ hoher Materialaufwand erforderlich ist, um durch ein vollständiges Ausfüllen der Poren der Auflageschicht eine sichere Dichtheit der Schicht zu erreichen. Infolge des bei einem Tränken vollständig in die Schicht eindringenden flüssigkeitsdichten Füllmaterials wird die Auflageschicht ziemlich hart und relativ steif. In Fällen, in denen der auszustattende Wegwerfartikel mit der Auflageschicht an dem Körper einer den Wegwerfartikel auf der Haut tragenden Person zu liegen kommt, wird dies als unangenehm empfunden; denn bei an dem Körper zu tragenden Wegwerfartikeln ist die direkt an der Haut anliegende poröse Auflageschicht aus weichem, angenehm zu tragendem Material gefertigt. Diese angenehmen Trageeigenschaften besitzt ein flüssigkeitsdichtes Füllmaterial naturgemäss nicht.

Bei einem weiteren, aus der US-PS 3,604,422 bekannten saugfähigen Wegwerfartikel kann auf die poröse Auflageschicht u.a. ein flüssigkeitsdichter Überzug aufgetragen werden. Bei dem Auftragen eines solchen Überzuges kommt es wie nach der US-PS 3,799,167 wiederum meist zu einem unerwünscht tiefen Eindringen des aufzutragenden flüssigkeitsdichten Materials. Unerwünscht ist ein zu tiefes Eindringen des die Sperrschicht bildenden Materials aus den bereits zu der oben zitierten US-PS 3,799,167 aufgezählten Gründen.

Von dem vorgenannten Stand der Technik ausgehend liegt der Erfindung die Aufgabe zugrunde, eine absolut dichte und mechanisch ausreichend feste Flüssigkeitssperrschicht mit flächenmässig beliebig vorgebbarer Ausdehnung unter Einsatz einer geringst möglichen Materialmenge zu schaffen. Dabei soll gleichzeitig sichergestellt sein, dass die einer Trägerperson zugewandte Seite der Auflageschicht absolut frei von dem die eigentliche Sperrschicht bildenden Material ist. Ausserdem soll durch die Flüssigkeitssperrschicht die Weichheit der Auflageschicht möglichst wenig beeinträchtigt werden.

Gelöst wird diese Aufgabe dadurch, dass die Auflageschicht des gattungsgemäßen saugfähigen Wegwerfartikels mit einer Flüssigkeitssperrschicht versehen ist, die aus einem heißsiegelfähigen, insbesondere thermoplastischen Material besteht, das in der Form einer vorgefertigten Dichtfolie unter vollflächiger physikalischer Bindung in beliebig vorbestimmbaren Bereichen nur geringfügig in die Oberfläche der porösen Auflageschicht eingreift, ohne diese Auflageschicht insgesamt auch nur annähernd vollständig zu durchdringen.

Der für die Ausbildung der Flüssigkeitssperrschicht angestrebte minimierte Materialaufwand wird dabei dadurch erreicht, dass die Sperrschicht eine vorgefertigte Folie aus einem klebefähigen Material ist, die als Ganzes unter lediglich leichtem vollflächigem Druck auf die Auflageschicht des Wegwerfartikels aufgebracht ist.

Während das Material einerseits vollflächig geringfügig in die Oberfläche der Auflageschicht eindringen soll, um zusammen mit dem Material der Auflageschicht den für eine ausreichende Festigkeit der Sperrschicht erforderlichen Materialverbund beider Schichten zu erreichen, soll es andererseits wiederum aus Gründen eines möglichst geringen Schichtmaterialeinsatzes nicht mehr als für die Erzielung des festigkeitsmässigen Verbundes unbedingt notwendig die unter der Oberfläche der Auflageschicht liegenden Freiräume ausfüllen.

Um dies optimal zu erreichen, weist die Dichtfolie in ihrem vorgefertigten Zustand zwei unterschiedlich gestaltete Oberflächen auf. Die die Auflageschicht nicht kontaktierende Oberfläche der Dichtfolie, die an dem Saugkörper des Wegwerfartikels zu liegen kommt, ist möglichst glatt ausgebildet, um auch noch bei geringster Dicke der Folie absolute Dichtheit zu gewährleisten. Die auf die Auflageschicht aufzulegende Oberfläche der Dichtfolie ist dagegen am besten von extrem zerklüfteter Struktur, da diese einen physikalisch hervorragenden Verbund zwischen Dichtfolie und Auflageschicht auch bereits dann garantiert, wenn das Dichtfolienmaterial mengenmässig insgesamt nur äusserst geringfügig in die Auflageschicht hineinragt.

Besonders vorteilhaft ist es, wenn die Dichtfolie nur über die Tiefe der zerklüfteten Oberfläche in die Auflageschicht eindringt. Dadurch ist es möglich, dass bei maximalen Eindringtiefen der höchsten Spitzen des zerklüfteten Oberflächenmaterials der Dichtfolie von etwa 15 μm bis 30 μm die über die gesamte Folienfläche gemittelte Eindringtiefe bei lediglich etwa 5 μm liegt.

Auf diese Weise können Dichtfolien verwendet werden, die - insbesondere, wenn sie aus einem auf Copolymere von L-Olefinen basierendem Material bestehen Flächengewichte von lediglich 20 bis 30 g/qm, insbesondere sogar nur 20 bis 25 g/qm, aufweisen.

Ein vorteilhaftes Verfahren zur Herstellung der erfindungsgemässen Artikel besteht darin, die Dichtfolie mit einer glatten und einer zerklüfteten Oberfläche dadurch zu erzeugen, dass das thermoplastische, die Flüssigkeitssperrschicht bildende Material in geschmolzenem Zustand auf die freie glatte Oberfläche einer rotierenden, insbesondere gekühlten Walze aufgetragen wird.

Um sicherzustellen, dass bei einem lediglich unter Transferdruck erfolgenden Aufbringen der Dichtfolie auf die Auflageschicht das Dichtfolienmaterial nicht bis über die gesamte Tiefe der Folie in die Auflageschicht eindringt, ist die Dichtfolie während des Auftragens ausschliesslich auf ihrer der Auflageschicht zugewandten Oberflächenseite in einem Tiefenbereich plastisch verformbar, der geringer als die Gesamtdicke der Dichtfolie ist. Die unterschiedliche plastische Verformbarkeit der Dichtfolie über deren Tiefe lässt sich während des Auftragens auf die Auflageschicht dadurch erreichen, dass die Temperatur über die Tiefe der Folie von der der Auflageschicht zugewandten Oberfläche aus derart abnimmt, dass in der freien glatten Oberfläche der Folie keine plastische Verformbarkeit mehr möglich ist.

Ein Verfahren, bei dem eine solche Temperierung der Dichtfolie während des Auftragens auf die Auflageschicht auf äusserst einfache und wirtschaftliche Weise erzielt werden kann, besteht darin, dass das heißsiegelfähige, insbesondere thermoplastische Material in geschmolzenem Zustand auf die Oberfläche einer rotierenden Walze aufgegeben wird, deren Temperatur niedriger als die Umgebungstemperatur ist, und dass die sich auf der Walze ausbildende Dichtfolie mit ihrer nicht an der Walze anliegenden freien Oberfläche direkt von der Walze auf die Auflageschicht aufgebracht wird.

Bei saugfähigen Wegwerfartikeln, die aus endlosem, einzelne in Abständen hintereinander liegende Saugkörper umhüllendem Bahnenmaterial stückweise abgelängt werden und nur in einzelnen sich quer zur Förderrichtung des Bahnenmaterials erstreckenden Bereichen (z.B. Taillenabschlussbereiche von Windeln) Dichtfolien aufweisen, die jeweils diskontinuierlich zugeführt werden, wird das die Dichtfolie erzeugende heißsiegelfähige, insbesondere thermoplastische, Material in geschmolzenem Zustand entsprechend diskontinuierlich auf eine rotierende Walze aufgegeben und von dort als Dichtfolieneinzelteil direkt auf das endlos ablaufende Auflageschicht-Bahnenmaterial übertragen.

Bei den erfindungsgemässen Flüssigkeitssperrschichten ist es auf recht einfache Weise möglich, den physikalischen Verbund zwischen Dichtfolie und Auflageschicht in einzelnen vorbestimmten Flächenabschnitten zu verstärken. Erwünscht können solche Verstärkungen z.B. bei Windeln in denjenigen Bereichen sein, an denen Klebestreifen zum Verschliessen der Windeln am Körper des Trägers anzubringen sind, oder im äussersten Randbereich der Windel, in dem die äussere Schutzfolie mit der Auflageschicht verbunden ist. Im zuletzt genannten Fall kann die Dichtfolie sogar als Klebemittel zur Erzielung des angestrebten Verbundes verwendet werden. Um dies bzw. auch nur eine nennenswerte Verstärkung des Auflageschichtmaterials zu erhalten, wird die Dichtfolie in dem betreffenden lokal begrenzten Bereich ggf. bis über ihre gesamte Tiefe plastisch eingestellt, wodurch sie die Auflageschicht beim Auftragen vollständig durchdringen kann. Bei vollständig durch die Auflageschicht hindurchgedrungenem Dichtfolienmaterial ist an all denjenigen Stellen, an denen die Auflageschicht direkt an der Schutzfolie anliegt, ein gemeinsamer fester Verbund erzielbar. Dadurch lässt sich z.B. die Ausreissfestigkeit der Klebeverschlußstreifen an den Windeln ganz erheblich erhöhen.

Die Zonen unterschiedlicher plastischer Verformbarkeit der Dichtfolie lassen sich bei einer Erzeugung der Dichtfolie auf einer rotierenden Walze und sich sofort daran anschliessender Übertragung auf den herzustellenden Artikel recht vorteilhaft dadurch erreichen, dass die Bereiche vollständiger plastischer Verformbarkeit der Dichtfolie durch geringere Kühlung der betreffenden Walzenbereiche erzeugt werden.

Abgesehen von einem vollständigen Eindringen des Dichtfolienmaterials in zu verstärkende Bereiche des erfindungsgemässen Artikels können diese Bereiche noch dadurch zusätzlich verstärkt werden, dass dort lokale Verdickungen der Dichtfolie vorgesehen werden. Die dickeren Dichtfolienbereiche können z.B. durch unterschiedlich dickes Auftragen des Dichtfolienausgangsmaterials auf die die Dichtfolie erzeugende rotierende Walze vorbestimmt werden.

Ein Ausführungsbeispiel der Erfindung ist in der Zeichnung dargestellt. Es zeigen:

| Fig. 1 | eine Windel in perspektivischer Ansicht mit einem aufgebrochenen Kantenbereich, |
|---|---|
| Fig. 2 | einen Teilschnitt nach Linie II-II, |
| Fig. 3 | den Teilschnitt nach Fig. 2 mit einer abgewandelten Anbindung der Dichtfolie an den Randbereich der Auflageschicht, |
| Fig. 4 | die perspektivische Ansicht einer Fertigungseinrichtung zur Herstellung einer Dichtfolie und deren Auftragung auf ein kontinuierlich zugeführtes endloses Bahnenmaterial, |
| Fig. 5 | einen Teilschnitt durch eine Dichtfolie nach Linie V-V. |

Eine Windel besteht aus einem Saugkörper 1, der auf einer Seite durch eine flüssigkeitsundurchlässige Schutzfolie 2 und auf der anderen Seite durch ein Vlies als poröse Auflageschicht 3 abgedeckt ist. Die Schutzfolie 2 und die Auflageschicht 3 sind an den Rändern der Windel miteinander verklebt.

Um im Taillenbereich der Windel ein Austreten von in dem Saugkörper befindlicher Flüssigkeit durch die Auflageschicht 3 hindurch zu vermeiden, ist dort eine Dichtfolie 4 jeweils als streifenförmig verlaufende Flüssigkeitssperrschicht vorgesehen.

Diese Dichtfolie 4 besteht aus dem thermoplastischen Material Copolymere von L-Olefinen. Sie wird in vorgefertigtem Zustand auf die Auflageschicht 3 aufgebracht.

Das Herstellen und anschliessende Aufbringen der Dichtfolie 4 auf die Auflageschicht 3 geschieht auf folgende Weise:
Auf eine rotierende gekühlte Walze 5 mit glatter Oberfläche wird das die Dichtfolie 4 bildende thermoplastische Material in geschmolzenem Zustand aus einer Spritzdüse 6 intermittierend aufgetragen.

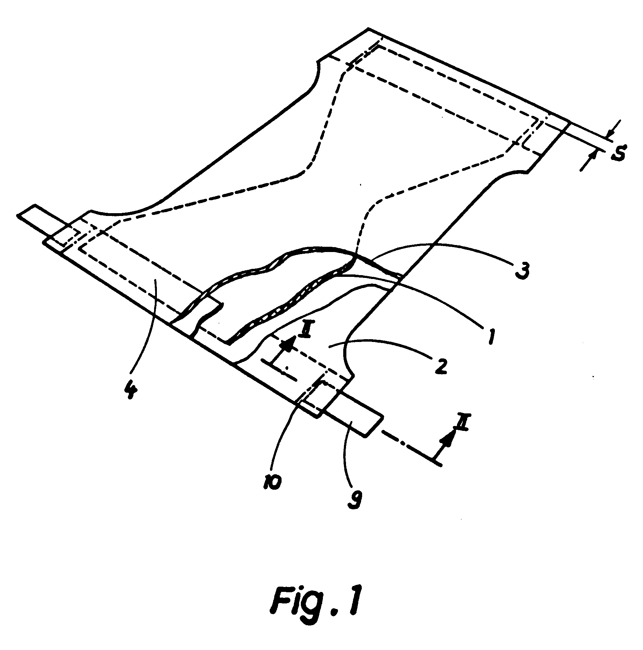

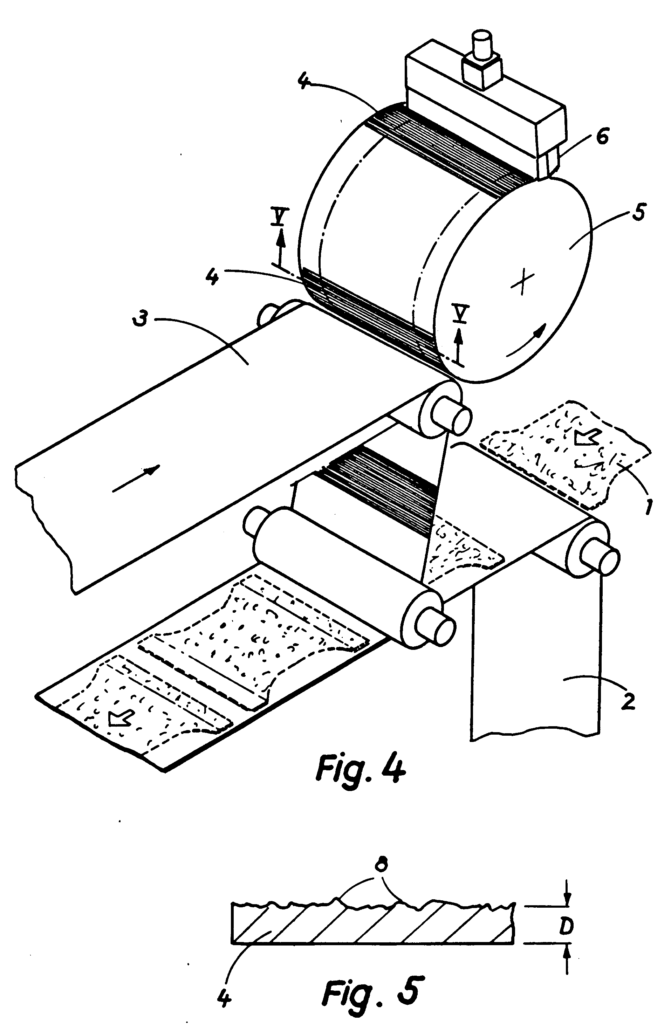

Die mittlere Dicke der auf der von der Walzenoberfläche abgewandten Oberfläche stark zerklüftet ausgebildeten Dichtfolie beträgt: $D = 22\ \mu m$ (die mittlere Dicke D ist in Fig. 5 dargestellt). Das Flächengewicht der Dichtfolie 4 beträgt 25 $g/m^2$. Die Kühlung der Walze 5 wird so eingestellt, dass im wesentlichen lediglich das in der zerklüfteten Oberfläche der Dichtfolie 4 liegende Material noch plastisch verformbar bleibt, während dies insbesondere an der an der Walzenoberfläche angrenzenden Dichtfolienoberfläche mit Sicherheit nicht mehr der Fall ist. In diesem Zustand werden die einzelnen Abschnitte der Dichtfolie 4 unter lediglich leichtem Druck auf die als Endlosbahnenware 7 zugeführte Auflageschicht 3 übertragen. Die so mit der Dichtfolie 4 verbundene Endlosbahnenware 7 wird sodann auf die ebenfalls als Endlosbahnenware zugeführte, den Saugkörper 1 in Abständen bereits enthaltende Schutzfolie 2 aufgebracht, wobei die Auflageschicht 3 und die Schutzfolie 2 an den Windelrändern miteinander verklebt werden. Die in den Tailenabschlussrändern der Windel zu liegen kommenden Dichtfolienabschnitte 4 können in den in Querrichtung zwischen den Saugkörpern 1 sich erstreckenden Bereichen S (Fig. 1) als Klebematerial verwendet werden. Das Verkleben kann dann dadurch erfolgen, dass in diesen Bereichen die aufeinanderliegenden Schichten einer lokalen Heißsiegelung unterzogen werden.

Die lediglich unter leichtem Druck auf die Auflageschicht 3 aufgebrachte Dichtfolie 4 dringt nur mit den Materialspitzen 8 in die Auflageschicht 3 ein. Auf diese Weise ist die angestrebte physikalische Bindung erreicht, die notwendig ist, um eine stabile Lage extrem dünner Dichtfolien in der Windel zu gewährleisten. Andererseits verbleibt stets ein während des Auftragevorgangs plastisch nicht verformbarer Dichtfolienbereich, der mit Sicherheit oberflächig geschlossen auf der Auflageschicht 3 ausgerichtet ist und für die erforderliche Dichtheit sorgt.

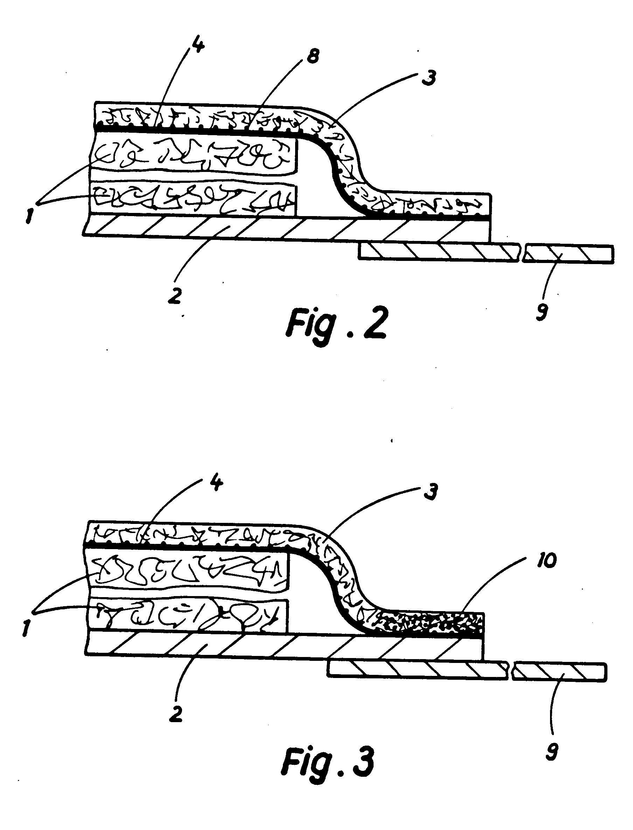

Für den Fall, dass ein Bereich der Windel, in dem Klebeverschlußstreifen 9 an die Schutzfolie 2 anzubringen sind, zusätzlich verstärkt werden soll, kann dort das Dichtfolienmaterial 4 vollständig in das Auflageschichtmaterial 3 eingedrückt werden. Dies ist in Fig. 3 dargestellt, in der das Dichtfolienmaterial 4 in dem betreffenden Randbereich 10 das poröse Auflageschichtmaterial 3 praktisch vollständig durchdringt. Diese unter den Klebeverschlußstreifen 9 liegenden Randbereiche 10 sind in Fig. 1 mit strichpunktierten Linien angedeutet.

Desgleichen sind in Fig. 4 auf der Walze 5 strichpunktierte Linien eingetragen, die dort jene Bereiche in entsprechender Weise andeuten. Der auf der Walze 5 sich von der jeweiligen strichpunktierten Linie bis zu dem Walzenrand jeweils erstreckende Dichtfolienrandbereich wird, wenn die Dichtfolie in diesen Bereichen in das Auflageschichtmaterial voll eindringen soll, weniger stark gekühlt. Dadurch bleibt während des Auftragens auf die Auflageschicht die Dichtfolie in diesen Bereichen über die gesamte Tiefe plastisch verformbar.

Wenn die Dichtfolie nicht direkt nach ihrer Erzeugung auf einer rotierenden Walze in einem Arbeitsgang auf die Auflageschicht 3 aufgetragen wird, muss sie erneut erwärmt werden, und zwar so, dass die vorstehend beschriebene unterschiedliche plastische Verformbarkeit bzw. lokale Nichtverformbarkeit gegeben ist.

**Patentansprüche**

1. Saugfähiger Wegwerfartikel, insbesondere Windel od.dgl., mit einem Saugkörper (1) einer die eine Seite des Saugkörpers (1) abdeckenden flüssigkeitsundurchlässigen Schutzfolie

4

(2), einer die gegenüberliegende Seite des Saugkörpers (1) abdeckenden porösen und flüssigkeitsdurchlässigen Auflageschicht (3), wobei die Schutzfolie (2) und die Auflageschicht (3) die Seitenränder des Saugkörpers (1) überragen und dort miteinander verbunden sind, und mit einer zumindest in einem Teil der Randbereiche des Wegwerfartikels zwischen der Schutzfolie (2) und der Auflageschicht (3) sich befindenden vorgefertigten Dichtfolie (4), wobei sich die Dichtfolie (4) über die ganze Länge des Randbereiches erstreckt und aus einem heißsiegelfähigen, insbesondere thermoplastischen Material besteht, **dadurch gekennzeichnet,** dass die eine Oberfläche der Dichtfolie (4) glatt und die andere zerklüftet ausgebildet ist und die Dichtfolie (4) nur mit den Spitzen der zerklüfteten Oberfläche mit der Auflageschicht (3) verbunden ist.

2. Wegwerfartikel nach Anspruch 1, dadurch gekennzeichnet, dass die Dichtfolie (4) im Mittel 5 $\mu$m und maximal 7 $\mu$m in die Auflageschicht (3) hineinragt.

3. Wegwerfartikel nach Anspruch 1 oder 2, dadurch gekennzeichnet, dass die Dichtfolie (4) aus Copolymeren von $\alpha$-Olefinen besteht und ein Flächengewicht von 20 bis 30 g/m$^2$, insbesondere 20 bis 25 g/m$^2$, aufweist.

4. Wegwerfartikel nach einem der vorhergehenden Ansprüche, dadurch gekennzeichnet, dass die Dichtfolie (4) eine unterschiedliche Dicke aufweist.

5. Verfahren zur Herstellung einer Dichtfolie (4) eines saugfähigen Wegwerfartikels nach einem der vorhergehenden Ansprüche, dadurch gekennzeichnet, dass das Material der Dichtfolie (4) in geschmolzenem Zustand auf die freie glatte Oberfläche einer rotierenden Kühlwalze (5) aufgetragen wird und das Material so weit abgekühlt wird, dass die auf der Walze (5) aufliegende Oberfläche der Dichtfolie (4) plastisch nicht mehr verformbar ist und die der Walze (5) abgekehrte Oberfläche der Dichtfolie (4) plastisch verformbar bleibt.

6. Verfahren nach Anspruch 5, dadurch gekennzeichnet, dass durch eine unterschiedliche Temperierung der Walze (5) die Dicke des plastischen Bereichs der Dichtfolie (4) eingestellt wird.

7. Verfahren nach Anspruch 5 oder 6, dadurch gekennzeichnet, dass die auf der eine Temperatur niedriger als die Erweichungstemperatur des thermoplastischen Materials aufweisenden Walze (5) sich ausbildende Dichtfolie (4) mit ihrer von der Walze (5) abgewandten Oberfläche direkt von der Walze (5) auf die Auflageschicht (3) aufgetragen wird.

8. Verfahren nach einem der Ansprüche 5 bis 7, dadurch gekennzeichnet, dass das thermoplastische Material diskontinuierlich auf die Walze (5) aufgetragen und von der Walze (5) auf eine die Auflageschicht (3) bildende Endlosbahn übertragen wird.

9. Verfahren nach einem der Ansprüche 5 bis 8, dadurch gekennzeichnet, dass das Material der Dichtfolie (4) in dem Bereich (10), der einen Klebeverschlußstreifen (9) zum Verschliessen eines als Windel ausgebildeten Wegwerfartikels aufweist, aufgetragen wird.

10. Verfahren nach Anspruch 9, dadurch gekennzeichnet, dass im Bereich der Klebeverschlußstreifen (9) mehr Material aufgetragen wird.

**Claims**

1. Disposable absorbent article, in particular baby's napkin or the like, with an absorbent body (1) of a liquid-impermeable protective sheet (2) which covers one side of said absorbent body (1), and a porous and liquid-permeable cover layer (3) which covers the opposite side of said absorbent body (1), said protective sheet (2) and said cover layer (3) projecting beyond the side edges of said absorbent body (1) and being bonded to each other there, and with a prefabricated impervious sheet (4) located between said protective sheet (2) and said cover layer (3) in at least a portion of the edge areas of said disposable article, said impervious sheet (4) extending over the entire length of said edge area and consisting of a heat-sealable, in particular thermoplastic, material, characterized in that the one surface of said impervious sheet (4) has a smooth structure and the other a coarse structure and said impervious sheet (4) is connected to said cover layer (3) with only the tips of said coarse surface.

2. Disposable article as defined in claim 1, characterized in that said impervious sheet (4) penetrates into said cover layer (3) to an average of 5 $\mu$m and to a maximum of 7 $\mu$m.

3. Disposable article as defined in claim 1 or 2, characterized in that said impervious sheet (4) consists of copolymers of $\alpha$-olefines and has an area weight of 20 to 30 g/m$^2$, in particular 20 to 25 g/m$^2$.

4. Disposable article as defined in one of the preceding claims, characterized in that said impervious sheet (4) differs in thickness.

5. Process for the manufacture of an impervious sheet (4) of a disposable absorbent article as defined in one of the preceding claims, characterized in that the material of said impervious sheet (4) is applied in the molten state to the free, smooth surface of a rotating cooled roll (5) and the material is cooled down to such an extent that the surface of said impervious sheet (4) resting on said roll (5) is no longer plastically deformable and the surface of said impervious sheet (4) facing away from said roll (5) remains plastically deformable.

6. Process as defined in claim 5, characterized in that the thickness of the plastic region of said impervious sheet (4) is set by varied tempering of said roll (5).

7. Process as defined in claim 5 or 6, characterized in that said impervious sheet (4) forming on said roll (5) whose temperature is lower than the softening temperature of said thermoplastic material is brought with its surface facing away from said roll (5) directly from said roll (5) onto said cover layer (3).

8. Process as defined in one of claims 5 to 7, characterized in that said thermoplastic material is dispensed in a discontinuous manner onto said roll (5) and is transferred from said roll (5) onto a continuous running web forming said cover layer (3).

9. Process as defined in one of claims 5 to 8, characterized in that the material of said impervious sheet (4) is applied in that area (10) in which there is an adhesive closure strip (9) for closing a disposable article in the form of a baby's napkin.

10. Process as defined in claim 9, characterized in that more material is applied in the area of said adhesive closure strips (9).

**Revendications**

1. Article absorbant jetable, notamment couche ou analogue, comportant un corps absorbant (1), une feuille protectrice (2) qui est imperméable aux liquides et recouvre un premier coté du corps absorbant (1), une couche de contact (3) poreuse et perméable aux liquides, laquelle recouvre le côté opposé du corps absorbant (1), la feuille protectrice (2) et la couche de contact (3) débordant et se réunissant l'une à l'autre au-delà des bords latéraux du corps absorbant (1), et une feuille d'étanchéité préfabriquée (4) se trouvant au moins dans une partie des zones marginales de l'article de table, entre la feuille protectrice (2) et la couche de contact (3), ladite feuille d'étanchéité (4) s'étendant sur toute la longueur de la zone marginale et étant constituée par un matériau notamment thermoplastique, scellable à chaud,
caractérisé
par le fait que la feuille d' étanchéité (4) est dotée d'une première face qui est lisse, son autre face étant fripée, cette feuille d'étanchéité (4) n'étant liée à la couche de contact (3) que par les pointes de la face fripée.

2. Article jetable selon revendication 1, caractérisé par le fait que la feuille d'étanchéité (4) pénètre en moyenne de 5 $\mu$m et au maximum de 7 $\mu$m dans la couche de contact (3).

3. Article jetable selon revendication 1 ou 2, caractérisé par le fait que la feuille d'étanchéité (4) est en copolymères de $\alpha$-oléfines et présente un poids surfacique de 20 à 30 g/m$^2$, notamment de 20 à 25 g/m$^2$.

4. Article jetable selon l'une des revendications précédentes, caractérisé par le fait que la feuille d'étanchéité (4) présente une épaisseur différente.

5. Procédé de fabrication d'une feuille d'étanchéité (4) d'un article absorbant jetable selon l'une des revendications précédentes, caractérisé par le fait que le matériau de la feuille d'étanchéité (4) est appliqué, à l'état fondu, sur la face lisse exposée d'un cylindre refroidisseur tournant (5) et est refroidi à un point tel que la face de' la feuille d'étanchéité (4) appliquée sur le cylidnre (5) ne soit plus déformable plastiquement et que la face de la feuille d'étanchéité (4) non en regard du cylindre (5) reste déformable plastiquement.

6. Procédé selon revendication 5, caractérisé par le fait que l'épaisseur de la zone plastique de la feuille d'étanchéité (4) est réglée en jouant sur la mise en température du cylindre.

7. Procédé selon revendication 5 ou 6, caractérisé par le fait que, par sa face non en regard du cylindre, la feuille d'étanchéité (4) se formant sur le cylindre (5) qui présente une température inférieure à la température d'amolissement du matériau thermoplastique est directement appliquée par le cylindre (5) sur la couche de contact (3).

8. Procédé selon l'une des revendications 5 à 7, caractérisé par le fait que le matériau thermoplastique est appliqué de manière discontinue sur le cylindre (5) et est transféré par celui-ci (5) sur une bande sans fin formant la couche de contact (3).

9. Procédé selon l'une des revendications 5 à 8, caractérisé par le fait que le matériau de la feuille d'étanchéité (4) est appliqué dans la région (10) qui présente une bande de fermeture collante (9) destinée à fermer un article jetable conçu-réalisé en tant que couche.

10. Procédé selon revendication 9, caractérisé par le fait que l'on applique davantage de matériau dans la région des bandes de fermeture collantes (9).

Fig.1

Fig . 2

Fig . 3

Fig. 4

Fig. 5